# EUROPÄISCHE PATENTSCHRIFT

(11) **EP 0 667 159 B1**
(45) Veröffentlichungstag und Bekanntmachung des Hinweises auf die Patenterteilung: **30.09.1998**
(21) Anmeldenummer: 95101954.6
(22) Anmeldetag: 14.02.1995
(51) Int. Cl.: A61K 31/505, A61K 47/02, A61K 9/08

(54) **Ampulle, die eine konzentrierte Injektionslösung von Alkalimetallsalzen von reduzierten Folaten enthält.**
Ampules containing a concentrated injectable solution of alcaline metal salts of reduced folates
Ampule contenant une solution injectable concentrée de sels de métaux alcalins de falates réduits

(30) Priorität: 14.02.1994 CH 432/94
(43) Veröffentlichungstag der Anmeldung: 16.08.1995
(73) Patentinhaber: Cerbios-Pharma S.A., CH-6917 Barbengo (CH)
(72) Erfinder: Buchs, Peter, CH-6927 Agra (CH); Marazza, Fabrizio, CH-6986 Novaggio (CH)
(74) Vertreter: Zink-Wild, Markus Peter

(56) Entgegenhaltungen:
- EP-A- 0 293 029
- EP-A- 0 401 895
- EP-A- 0 416 232
- WO-A-90/10460
- US-A- 2 695 860
- US-A- 5 124 452
- REYNOLDS J.E.F. 'Martindale , The extra Pharmacopoeia' 1993 , THE PHARMACEUTICAL PRESS , LONDON 186195 * Seite 1040 - Seite 1041 *
- Hagers Handbuch der Pharmazeutischen Praxis. seite 358

## Beschreibung

Die Vorliegende Erfindung betrifft eine Ampulle, in die eine konzentrierte, stabile Injektionslösung abgefüllt ist, und ein Verfahren zur Herstellung dieser Ampulle.

N(5)-Formyl-5,6,7,8-tetrahydrofolsäure, auch Folinsäure genannt, wird in der Form des Calciumsalzes (Calcium-Leucovorin USP) als Mittel in der Krebs-Chemotherapie verwendet.

Dieses Mittel wird sowohl für Hilfeleistungen (rescue agent) nach Behandlung mit hohen Dosen des cytostatischen Mittels Methotrexat als auch in Kombination mit dem Antikrebsmittel 5-Fluorouracil (5-FU) verwendet.

Siehe zum Beispiel:
1. The clinical pharmacology of methotrexate, J.H. Schornagel und J.G. McVie, Cancer Treatment Reviews (1983), 10, 53.
2. Die biochemische Modulation von 5-FU durch Leucovorin, M. Borner, K. Brunner, Schweiz. Med. Wschr., (1991), 121, 1149.

Folinsäure wird auch verwendet für die Behandlung von megaloblastischen Anämien und Dihydropteridin Reduktase Defizienz.

Diese therapeutischen Verwendungen von Folinsäure erfordern manchmal die intravenöse Verabreichung von hohen Dosen, beispielsweise 100 bis 200 mg/m² Körperoberfläche.

Weil Calcium-Leucovorin in Wasser relativ schlecht löslich ist (etwa 1 Gew.-% bei Raumtemperatur) besteht ein starker Bedarf an einer Formulierung, welche eine höhere Konzentration des aktiven Prinzipes enthält.

Mit solchen hohen Konzentrationen kann vermieden werden, dass unannehmbar hohe Dosen an Lösung injiziert werden müssen.

Wenn, gemäss einer privaten Mitteilung von Dr. C. Young vom Memorial Sloan Kettering Cancer Center in New York, 5-FU und Leucovorin in einer einzigen Infusion miteinander kombiniert sind, im Sinne einer Behandlungsvorschrift mit 5-FU, dann trägt die Anwesenheit von Calciumionen zur Blockierung des Katheters bei.

Wenn der Gehalt an Leucovorin nicht stark reduziert wird, dann wird die Blockierung des Katheters durch einen Niederschlag zu einem sich wiederholenden Problem.

Es ist berichtet worden, dass der Niederschlag Calciumcarbonat ist, wahrscheinlich gebildet durch die Reaktion von Calcium mit Plasma-bicarbonat bei oder in der Nähe der Katheterspitze.

Die therapeutische Verwertung von Leucovorin könnte erhöht werden durch die Verfügbarkeit eines besser löslichen calciumfreien Leucovorin-Salzes.

Die US 5 124 452 betrifft chemische Herstellungsverfahren von N(5)-CH₃-THF Salzen.

In Anspruch 1 wird die Herstellung des Na-Salzes, in Anspruch 9 die Herstellung des Ca-Salzes und in Anspruch 13 ganz generell die Herstellung von Erdalkalimetallsalzen beschrieben.

In einem Nebenaspekt werden in diesem Dokument Formulierungen genannt; siehe Spalten 7 und 8.

Sowohl bei der oralen als auch bei der injizierbaren Formulierung müssen zwingend ein Stabilisator und ein Arzneimittelträger anwesend sein.

Gemäss der Aussage in Spalte 7, Zeilen 26 bis 35 müssen 5 bis 50 % an Stabilisator anwesend sein.

Eine flüssige Formulierunmg ist in diesem Dokument nicht genannt; es werden hierin nur feste Formulierungen erwähnt.

Die in Spalte 7 beschriebene "injectable formulation" ist ein festes, lyophilisiertes Präparat, welches nebst dem "lyophilisation-carrier" noch einen Stabilisator enthält.

Diese als Festkörper vorliegende "injectable formulation" kann beliebig verdünnt werden.

In Beispiel 6 von EP 0 293 029 wird aus dem Calcium-Salz der Folinsäure die entsprechende freie Säure hergestellt.

In Beispiel 5 dieses Dokumentes wird die Herstellung eines Festkörpers beschrieben.

Ueber die Stabilität der für die Herstellung dieser Festkörper benötigten Lösungen wird in diesem Dokument nichts ausgesagt.

Dieses Dokument ist ein "Verfahrens- und Stoffpatent"; von einer konzentrierten stabilen Lösung wird hierin nichts erwähnt.

Es ist dem Fachmann bekannt, dass der Ersatz von Ca²⁺-Ionen durch Na⁺ oder K⁺-Ionen im Falle von Salzen von reduzierten Folaten nichts an der therapeutischen Wirkung des aktiven Prinzipes ändert.

Auf die Nachteile der Calciumsalz-Lösungen von reduzierten Folaten ist bereits weiter oben hingewiesen worden.

Im Stand der Technik sind bis jetzt zwei Injektionslösungen auf der Basis von Folaten bekannt:
A. In der Europäischen Patentanmeldung Nr. 90 112 426.3, Veröffentlichungs-Nr. 0 416 232 A2 wird eine stabile injizierbare pharmazeutische Formulierung für Folsäure und Leucovorin beschrieben.
   Diese Formulierung enthält nebst einem wasserlöslichen, pharmazeutisch annehmbaren Salz von Folsäure oder Leucovorin verschiedene Stabilisierungsmittel, wie etwa Benzylalkohol, Tromethamin und Monothioglycerol.
   Diese Formulierung kann maximal 2,5 % an Calcium-Leucovorin enthalten.
   Die verschiedenen Stabilisierungsmittel sind sicherlich ein Nachteil, weil einzelne Komponenten nicht in allen Pharmacopeias als pharmakologisch annehmbar in injizierbaren Präparaten beschrieben sind.
B. In der Europäischen Patentanmeldung Nr. 90 201 353.1, Veröffentlichungs-Nr. 0 401 895 A1 wird eine wässrige Calcium-Folinatlösung beschrieben, welche bei Kühlschranktemperatur stabil ist. Diese Lösung muss ein Komplexierungsmittel für Calcium enthalten.

Als einziges Komplexierungsmittel wird Ethylendiamin-tetraacetat (EDTA) genannt.

Diese Lösung kann maximal 2,7 % an Calcium-Folinat enthalten.

EDTA und ähnliche Komplexierungsmittel sind nicht annehmbar als Komponenten in einer Injektionslösung.

Es ist ein Ziel der vorliegenden Erfindung, die oben genannten Nachteile zu überwinden.

Es ist ein weiteres Ziel der vorliegenden Erfindung, eine konzentrierte, stabile Lösung auf der Basis von Folaten zur Verfügung zu stellen, welche weder ein Stabilisierungsmittel noch ein Komplexierungsmittel enthält.

Es wurde nun völlig überraschend gefunden, dass die Lösungen, die sich in der erfindungsgemässen Ampulle befinden, bei Temperaturen von 0°C bis 5°C ohne die Hinzugabe von Stabilisierungsmitteln und/oder Komplexierungsmitteln während wenigstens 12 Monaten stabil sind.

Die konzentrierte, stabile Injektionslösung ist dadurch gekennzeichnet, dass sie nebst Wasser entweder Natrium-Leucovorin oder Kalium-Leucovorin oder Natrium-N(5)-Methyl-5,6,7,8-tetrahydrofolsäure oder Kalium-N(5)-Methyl-5,6,7,8-tetrahydrofolsäure enthält, aber weder ein Stabilisierungsmittel noch ein Komplexierungsmittel enthält.

Vorzugsweise enthält diese Injektionslösung von 2 bis 15 Gew.-%, insbesondere von 2 bis 6 Gew.-%, vorzugsweise 5 Gew.-%, Natrium-Leucovorin oder Kalium-Leucovorin oder Natrium-N(5)-Methyl-5,6,7,8-tetrahydrofolsäure oder Kalium-N(5)-Methyl-5,6,7,8-tetrahydrofolsäure und weist einen pH-Wert im Bereich von 7,5 bis 8,5, insbesondere 7,9 bis 8,1, vorzugsweise 8,0, auf.

Das Verfahren zur Herstellung einer erfindungsgemässen Ampulle, ist dadurch gekennzeichnet, dass dass man Folinsäure oder N(5)-Methyl-5,6,7,8-tetrahydrofolsäure in Wasser, welches entgast und für die Herstellung von Injektionslösungen annehmhar ist, bei Raumtemperatur unter einer Inertgasatmosphäre suspendiert, dann eine wässrige Lösung von Natrium- oder Kalium-hydroxid, -hydrogencarbonat oder -carbonat portionenweise so lange hinzugibt, bis eine klare Lösung entstanden ist, welche den jeweils gewünschten pH-Wert aufweist, die erhaltene Lösung einer sterilen Filtration unterwirft, und die erhaltene sterile Lösung unter einer Inertgasatmosphäre in Ampullen abfüllt.

Die Lösungen können verwendet werden
- zur Herstellung eines Arzneimittels für Hilfeleistungen - rescue agent - nach Behandlung mit hohen Dosen an Methotrexat, oder
- zur Herstellung eines Arzneimittels, welches kombiniert ist mit 5-Fluorouracil, oder
- zur Herstellung eines Arzneimittels für die Behandlung von megaloblastischen Anämien und Dihydropteridin Reduktase Defizienz.

Bevorzugte Ausführungsformen der vorliegenden Erfindung sind in den abhängigen Ansprüchen definiert.

Das nachfolgende Beispiel illustriert die vorliegende Erfindung.

### Beispiel

200,7 g Folinsäure mit einem Wassergehalt von 10,2 Gew.-% - beispielsweise hergestellt gemäss E. Khalifa, A.N. Ganguly, J.H. Bieri und M. Viscontini, Helv. Chim. Acta, Vol 63, 2554, (1980) - wurden in 2,5 Liter entgastem, sterilem Wasser unter einer Stickstoffatmosphäre und unter Rühren bei Raumtemperatur suspendiert.

Anschliessend wurde unter Rühren eine 10%-ige wässrige Natriumhydroxidlösung hinzugetropft, bis eine klare Lösung entstanden war, welche einen pH-Wert von 8,0 hatte.

Die erhaltene klare Lösung wurde durch Hinzugabe von entgastem, sterilem Wasser auf ein Volumen von 3,6 Liter verdünnt.

Diese verdünnte Lösung wurde einer sterilen Filtration (Porengrösse: 0,2 Mikrometer) unterworfen.

Das erhaltene sterile Filtrat wurde unter einer Stickstoffatmosphäre in Glasampullen abgefüllt.

Diese Ampullen wurden in einem Kühlschrank bei einer Temperatur von 4°C gelagert.

Dabei wurden für die in die Ampullen abgefüllte Lösung die folgenden Stabilitätsdaten erhalten:

| Zeit (Monate) | Gehalt in % (HPLC-Analyse) |
|---|---|
| 0 | 100 |
| 3 | 99,0 |
| 6 | 96,8 |
| 12 | 95,2 |

Nach 12 Monaten war die Lösung immer noch klar; Niederschläge konnten keine festgestellt werden.

## Patentansprüche

1. Ampulle, dadurch gekennzeichnet, daß in sie eine konzentrierte, stabile Injektionslosung abgefüllt ist, welche nebst Wasser entweder Natrium-Leucovorin oder Kalium-Leucovorin oder Natrium-N(5)-Methyl-5,6,7,8-tetrahydrofolsäure oder Kalium-N(5)-Methyl-5,6,7,8-tetrahydrofolsäure enthält, aber weder ein Stabilisierungsmittel noch ein Komplexierungsmittel enthält, und in ihrem Innenraum eine Inertgasatmosphäre, insbesondere eine Stickstoffatmosphäre, hat.

2. Ampulle nach Anspruch 1, dadurch gekennzeichnet, daß die Injektionslösung von 2 bis 15 Gew.-%, insbesondere von 2 bis 6 Gew.-%, vorzugsweise 5 Gew.-%, Natrium-Leucovorin oder Kalium-Leucovorin oder Natrium-N(5)-Methyl-5,6,7,8-tetrahydrofolsäure oder Kalium-N(5)-Methyl-5,6,7,8-tetrahydrofolsäure enthält.

3. Ampulle nach einem der Ansprüche 1 bis 2, dadurch gekennzeichnet, daß die Injektionslosung einen pH-Wert im Bereich von 7,5 bis 8,5, insbesondere 7,9 bis 8,1, vorzugsweise 8,0, aufweist.

4. Verfahren zur Herstellung einer Ampulle nach einem der Ansprüche 1 bis 3, dadurch gekennzeichnet, daß man Folinsäure oder N(5)-Methyl-5,6,7,8-tetrahydrofolsäure in Wasser, welches entgast und für die Herstellung von Injektionslösungen annehmbar ist, bei Raumtemperatur unter einer Inertgasatmosphäre suspendiert, dann eine wässrige Lösung von Natrium- oder Kalium-hydroxid, -hydrogencarbonat oder -carbonat portionenweise so lange hinzugibt, bis eine klare Lösung entstanden ist, welche den jeweils gewünschten pH-Wert aufweist, die erhaltene Lösung einer sterilen Filtration unterwirft, und die erhaltene sterile Lösung unter einer Inertgasatmosphäre in Ampullen abfüllt.

5. Verfahren nach Anspruch 4, dadurch gekennzeichnet, daß die genannte Injektionslösung von 2 bis 15 Gew.-%, insbesondere von 2 bis 6 Gew.-%, vorzugsweise 5 Gew.-%, Natrium-Leucovorin oder Kalium-Leucovorin oder Natrium-N(5)-Methyl-5,6,7,8-tetrahydrofolsäure oder Kalium-N(5)-Methyl-5,6,7,8-tetrahydrofolsäure enthält.

6. Verfahren nach einem der Ansprüche 4 bis 5, dadurch gekennzeichnet, dass die genannte Injektionslösung einen pH-Wert im Bereich von 7,5 bis 8,5, insbesondere 7,9 bis 8,1, vorzugsweise 8,0, aufweist.

## Claims

1. Ampule, characterized in that it is filled into it a concentrated, stable injection solution, which contains beside water either sodium-leucovorin or potassium-leucovorin or sodium-N(5)-methyl-5,6,7,8-tetrahydrofolic acid or potassium-N(5)-methyl-5,6,7,8-tetrahydrofolic acid, but which contains neither a stabilizer nor a complexing agent, and having in its interior an inert gas atmosphere, especially a nitrogen atmosphere.

2. The ampule according to claim 1, characterized in that the injection solution contains from 2 to 15 % by weight, especially from 2 to 6 % by weight, preferably 5 % by weight, of sodium-leucovorin or potassium-leucovorin or sodium-N(5)-methyl-5,6,7,8-tetrahydrofolic acid or potassium-N(5)-methyl-5,6,7,8-tetrahydrofolic acid.

3. The ampule according to one of claims 1 to 2, characterized in that the injection solution has a pH-value in the range from 7.5 to 8.5, especially 7.9 to 8.1, preferably 8.0.

4. A process for the preparation of an ampule according to one of claims 1 to 3, characterized in that folinic acid or N(5)-methyl-5,6,7,8-tetrahydrofolic acid is suspended in water, which is degassed and which is acceptable for the preparation of injection solutions, at room temperature under an inert gas atmosphere, then is added an aqueous solution of sodium- or potassium-hydroxide, -hydrogen carbonate or -carbonate in portions during such a long time until a clear solution is formed, which has the at times desired pH-value, subjecting the obtained solution to a sterile filtration, and filling the obtained sterile solution under an inert gas atmosphere into ampules.

5. The process according to claim 4, characterized in that said injection solution contains from 2 to 15 % by weight, especially from 2 to 6 % by weight, preferably 5 % by weight, of sodium-leucovorin or potassium-leucovorin or sodium-N(5)-methyl-5,6,7,8-tetrahydrofolic acid or potassium-N(5)-methyl-5,6,7,8-tetrahydrofolic acid.

6. The process according to one of claims 4 to 5, characterized in that said injection solution has a pH-value in the range from 7.5 to 8.5, especially from 7.9 to 8.1, preferably 8.0.

## Revendications

1. Ampoule caractérisée en ce qu'on y a transvasé une solution concentrée stable pour injection qui contient, outre de l'eau, soit de la leucovorine sodique, soit de la leucovorine potassique, soit de l'acide N(5)-méthyl-5,6,7,8-tétrahydrofolique sous forme du sel de sodium, soit de l'acide N(5)-méthyl-5,6,7,8-tétrahydrofolique sous forme du sel de potassium, mais qui ne contient ni un agent de stabilisation ni un agent de complexation, et qui possède dans son espace interne une atmosphère de gaz inerte, en particulier une atmosphère d'azote.

2. Ampoule selon la revendication 1, caractérisée en ce que la solution pour injection contient, a concurrence de 2 à 15% en poids, en particulier de 2 à 6% en poids, de préférence à concurrence de 5% en poids, de la leucovorine sodique ou de la leucovorine potassique ou de l'acide N(5)-méthyl-5,6,7,8-tétrahydrofolique sous forme du sel de sodium ou de l'acide N(5)-méthyl-5,6,7,8-tétrahydrofolique sous forme du sel de potassium.

3. Ampoule selon l'une quelconque des revendications 1 à 2, caractérisée en ce que la solution pour injection présente une valeur de pH dans le domaine de 7,5 à 8,5, en particulier de 7,9 à 8,1, de préférence de 8,0.

4. Procédé pour la préparation d'une ampoule selon l'une quelconque des revendications 1 à 3, caractérisé en ce qu'on met en suspension de l'acide folinique ou de l'acide N(5)-méthyl-5,6,7,8-tétrahydrofolique dans de l'eau qui a été soumise à une dégazéification et qui est acceptable pour la préparation de solutions pour injection à la température ambiante et sous l'atmosphère d'un gaz inerte; ensuite, on y ajoute par portions une solution aqueuse d'hydroxyde, d'hydrogénocarbonate ou de carbonate de sodium ou de potassium jusqu'à ce que l'on obtienne une solution claire qui présente la valeur de pH respectivement désirée; on soumet la solution obtenue à une filtration dans des conditions stériles; et on transvase la solution stérile obtenue dans des ampoules sous l'atmosphère d'un gaz inerte.

5. Procédé selon la revendication 4, caractérisé en ce que la solution mentionnée pour injection contient, à concurrence de 2 à 15% en poids, en particulier de 2 à 6% en poids, de préférence à concurrence de 5% en poids, de la leucovorine sodique ou de la leucovorine potassique ou de l'acide N(5)-méthyl-5,6,7,8-tétrahydrofolique sous forme du sel de sodium ou de l'acide N(5)-méthyl-5,6,7,8-tétrahydrofolique sous forme du sel de potassium.

6. Procédé selon l'une quelconque des revendications 4 à 5, caractérisé en ce que la solution pour injection mentionnée présente une valeur de pH dans le domaine de 7,5 à 8,5, en particulier de 7,9 à 8,1, de préférence de 8,0.
